# EUROPÄISCHE PATENTSCHRIFT

(11) **EP 2 082 697 B1**
(45) Veröffentlichungstag und Bekanntmachung des Hinweises auf die Patenterteilung: **09.02.2011**
(21) Anmeldenummer: 08101001.9
(22) Anmeldetag: 28.01.2008
(51) Int. Cl.: A61B 17/70

(54) **Pedikelschraube mit einer Verschlusseinrichtung**
Pedicular screw with a locking device
Vis pédiculaire dotée d'un dispositif de fermeture

(43) Veröffentlichungstag der Anmeldung: 29.07.2009
(73) Patentinhaber: Spinelab AG, 6304 Zug (CH)
(72) Erfinder: Zehnder, Thomas, 8806, Bäch (CH); Braunschweiler, Reto, 8413, Neftenbach (CH); White, Patrick, West Chester, PA 19382 (US); Chenaux, Fabrice, Exton, PA 19341 (US)
(74) Vertreter: BOVARD AG

(56) Entgegenhaltungen:
- EP-A- 1 759 646
- EP-A- 1 815 812
- DE-U1- 9 403 231
- US-A- 5 667 508

## Beschreibung

Die vorliegende Erfindung bezieht sich auf eine Pedikelschraube mit einer Verschlusseinrichtung zur Befestigung eines Stabes zur Stabilisierung der Wirbelsäule, umfassend einen Einschraubteil, einen am Einschraubteil angebrachten Kopfteil, eine im Kopfteil angeordnete U-förmige Ausnehmung, die durch die inneren Oberflächen zweier Schenkel und einer die beiden inneren Oberflächen verbindenden Grundfläche gebildet ist, in welche Ausnehmung der Stab einsetzbar und durch die Verschlusseinrichtung gehalten ist, welcher Stab mit im Wesentlichen quer zur Stabachse verlaufenden Rippen und Rillen und welche Grundfläche mit diesen entsprechenden Rippen und Rillen ausgestattet sind.

Derartige Pedikelschrauben mit einer Verschlusseinrichtung zur Befestigung eines Stabes sind in vielfältiger Weise aus dem Stand der Technik bekannt. Diese dienen zur Stabilisierung von Wirbelsäulen von Patienten, welche Wirbelsäulen starke Schäden aufweisen. Zur Stabilisierung dieser Wirbelsäule wird in eine Anzahl von Wirbelkörpern jeweils eine Pedikelschraube eingeschraubt, in die Kopfteile dieser Pedikelschrauben wird eine Stange eingelegt, welche dann jeweils mit der entsprechenden Pedikelschraube verbunden wird, wozu Verschlusseinrichtungen verwendet werden. Es können mit derartigen Pedikelschrauben und eingelegter Stange im Wesentlichen zwei unterschiedliche Stabilisierungsformen erreicht werden, abhängig davon, welche Art von Stange einsetzt. Beim Einsatz einer starren Stange erreicht man eine Versteifung der betroffenen Wirbelkörper, für eine stützende Stabilisierung der Wirbelkörper kann eine elastische Stange eingesetzt werden, durch diese erreicht man, dass zwischen den einzelnen Wirbelkörpern eine gewisse Beweglichkeit zugelassen wird.

Unabhängig davon, welches System eingesetzt wird, ist eine optimale Verbindung zwischen eingesetzter Stange und Pedikelschraube anzustreben, was durch die eingesetzten Verschlusseinrichtungen erreicht werden soll.

Aus der EPB 1 119 304 ist eine Vorrichtung zur Befestigung von Wirbelsäulenstangen bekannt, welche aus einer Pedikelschraube besteht, die einen Kopfteil mit einer U-förmigen Ausnehmung aufweist, in welche die zu fixierende Stange eingesetzt wird. Als Verschlusseinrichtung dient ein verdrehbares Element mit zwei einander gegenüber liegenden, seitlich vorstehenden Nocken, die eine schraubenförmige Neigung aufweisen. Diese Nocken gelangen durch Verdrehen dieses Drehelementes in entsprechende schlitzförmige Ausnehmungen der beiden Schenkel der U-förmigen Ausnehmung. Durch Verdrehen dieses Drehelements wird die Stange klemmend gehalten. Um ein Lösen dieses Drehelements vermeiden zu können, ist die Spannfläche, die gegen die Stange gerichtet ist, mit querverlaufenden Ausnehmungen versehen, die bei Erreichen der Klemmposition in die Oberfläche der Stange einklinken sollen.

Diese Einrichtung ist einfach zu handhaben, es dürfte aber nicht unproblematisch sein, mit den vorgegebenen Positionen der Ausnehmungen, die in die Stange einklinken sollen, die richtige Spannkraft erhalten zu können, damit die Stange in optimaler Weise in der Pedikelschraube festgeklemmt wird.

Es sind auch Einrichtungen bekannt, bei welchen zwischen der Pedikelschraube und dem Stab eine formschlüssige Verbindung erhalten wird, indem die Aufnahme in der Pedikelschraube für den Stab und der Stab selbst mit jeweils entsprechenden Rippen und Rillen ausgestattet sind, die quer zur Stabrichtung verlaufen, und die ineinander greifen. Bei diesen Vorrichtungen ist es nicht zwingend erforderlich, dass der Stab in der Pedikelschraube durch eine Klemmkraft gehalten wird, es kann ausreichend sein, dass die Pedikelschraube mit einer Verschlusseinrichtung verschlossen wird, die mit einer Klinkvorrichtung ausgestattet ist, welche mit Klinken versehen ist, die im verschlossenen Zustand in entsprechende Ausnehmungen der Pedikelschraube einklinken und der Stab entsprechend gehalten wird.

Derartige Vorrichtungen sind sehr einfach zu handhaben, im praktischen Einsatz muss aber sehr genau überprüft werden, ob die entsprechenden Klinken vollumfänglich in die jeweiligen Ausnehmungen der Pedikelschraube eingeklinkt sind.

Aus der EP 1 815 812 A ist eine weitere Pedikelschraube bekannt, aus der der Gegenstand des Anspruchs 1 abgeleitet wird.

Die Aufgabe der vorliegenden Erfindung besteht somit darin, eine Pedikelschraube mit einer Verschlusseinrichtung zur Befestigung eines Stabes zur Stabilisierung der Wirbelsäule zu schaffen, welche einfach zu handhaben ist und mit welcher gewährleistet ist, dass im verschlossenen Zustand die Verbindung zwischen Verschlusseinrichtung und Pedikelschraube optimal ist.

Erfindungsgemäss erfolgt die Lösung dieser Aufgabe dadurch, dass die Verschlusseinrichtung ein Tragelement umfasst, welches zwischen die beiden Schenkel einsetzbar ist, dass am Tragelement zwei Schiebeteile verschiebbar angeordnet sind, welche über einen Antriebsmechanismus von einer gegeneinander geschobenen Position in eine auseinander geschobene Position bringbar ist, in welcher auseinander geschobenen Position die beiden voneinander abgewandten Endbereiche der beiden Schiebeteile bei zwischen die Schenkel eingesetztem Zustand des Tragelements in jeweils eine Ausnehmung der beiden Schenkel hineinragen und die Verschlusseinrichtung mit der Pedikelschraube verriegelt ist.

Durch Betätigen des Antriebsmechanismus beim Verschliessen der Verschlusseinrichtung zum Befestigen eines Stabes in einer Pedikelschraube werden die beiden Schiebeteile zwangsweise in die beiden Ausnehmungen an der Pedikelschraube eingeführt, eine sichere Verriegelung ist dadurch gewährleistet.

In vorteilhafter Weise besteht der Antriebsmechanismus aus einem drehbaren Teil, der im Tragelement um eine senkrecht zur Verschieberichtung der beiden Schiebeteile stehende Achse drehbar angeordnet ist, welcher mit zwei parallel zur Achse ausgerichteten Zapfen ausgestattet ist, welche jeweils mit einer an jedem Schiebeteil angebrachten Steuerkurve zusammen wirken. Dadurch wird ein einfacher mechanischer Aufbau erreicht.

Die Schiebeteile und der drehbare Teil sind in das Tragelement eingelegt, auf das Tragelement ist ein Abschlussdeckel aufgesetzt und mit diesem verbunden, welcher mit einer Bohrung versehen ist, in welcher der drehbare Teil drehbar gelagert ist, wodurch in vorteilhafter Weise erreicht wird, dass die Verschlusseinrichtung als Einheit aufgebaut ist, die in einfacher Weise gehandhabt werden kann.

Eine weitere vorteilhafte Ausgestaltung der Erfindung besteht darin, dass zwischen drehbarem Teil und Tragelement Einrastelemente angeordnet sind, welche die beiden Schiebeteile in der auseinander geschobenen Position in einer Einrastlage festhalten. Durch das Einrasten der Einrastelemente wird einerseits der die Verschlusseinrichtung in die Pedikelschraube einsetzenden Person angezeigt, dass die Verschlusseinrichtung in optimaler Weise in die Pedikelschraube eingesetzt ist, andererseits ist die Lage der beiden sich in der auseinander geschobenen Position befindenden Schiebeteile gesichert, ein selbsttätiges Verschieben dieser beiden Schiebeteile wird dadurch vermieden.

In vorteilhafter Weise sind die Einrastelemente jeweils aus einem federnden Arm gebildet, dessen eines Ende am drehbaren Teil befestigt ist und an dessen federndem freien Ende ein Nocken angebracht ist, welcher in der Einrastlage in jeweils eine entsprechende am Tragelement angebrachte Ausnehmung einrastet. Dadurch wird neben einer optimalen Funktion auch ein einfacher und kompakter Aufbau erreicht.

Zur Betätigung des drehbaren Teils ist dieser mit einer profilierten Vertiefung ausgestattet, in welche ein mit einer entsprechenden profilierten Form versehenes Drehwerkzeug eingesteckt werden kann.

In vorteilhafter Weise sind die voneinander abgewandten Endbereiche der Schiebeteile mit Anschrägungen versehen, wodurch ein störungsfreies Einfahren der beiden Schiebeteile in die beiden Ausnehmungen der beiden Schenkel der Pedikelschraube gewährleistet ist.

In vorteilhafter Weise weist die dem Stab zugewandte Oberfläche des Tragelements eine der Staboberfläche angepasste Form auf und ist diese Oberfläche mit entsprechenden Rippen und Rillen ausgestattet, wodurch eine optimale Fixierung des Stabes in der Pedikelschraube erreichbar ist.

In vorteilhafter Weise ist das Tragelement an seinen Eckbereichen mit Führungsrippen ausgestattet, mittels welchen das Tragelement geführt auf den Kopfteil, der mit Führungsflächen versehen ist, aufsetzbar ist. Dadurch wird das Aufsetzen der Verschlusseinrichtung auf die Pedikelschraube vereinfacht.

Eine weitere vorteilhafte Ausgestaltung der Erfindung besteht darin, dass die Pedikelschraube einen in ihre U-förmige Ausnehmung einsetzbaren und durch die Verschlusseinrichtung gehaltenen Stab aufweist, der aus einem elastischen Material, insbesondere aus einem biokompatiblen Kunststoff auf der Basis von Polyurethan gebildet ist, wodurch eine stützende Stabilisierung der Wirbelkörper erreicht wird.

Eine Ausführungsform der Erfindung wird nachfolgend anhand der beiliegenden Zeichnung beispielhaft näher erläutert.

Es zeigt,
Fig. 1 in räumlicher Darstellung eine Pedikelschraube mit einer darauf aufgesetzten Verschlusseinrichtung mit einem Stab;
Fig. 2 in räumlicher Darstellung den Kopfteil der Pedikelschraube und die einzelnen Elemente der Verschlusseinrichtung in auseinander gezogener Lage;
Fig. 3 eine Schnittdarstellung durch die Pedikelschraube mit aufgesetzter Verschlusseinrichtung in verriegelter Position;
Fig. 4 in räumlicher Darstellung eine Ansicht auf das Tragelement der Verschlusseinrichtung mit eingesetztem drehbaren Teil und einem der beiden Schieber;
Fig. 5 eine Draufsicht im Schnitt auf die Verschlusseinrichtung mit den beiden Schiebeteilen in der gegeneinander geschobenen Position;
   und
Fig. 6 eine Draufsicht im Schnitt auf die Verschlusseinrichtung mit auseinander geschobenen Schiebeteilen und eingerasteten Einrastelementen.

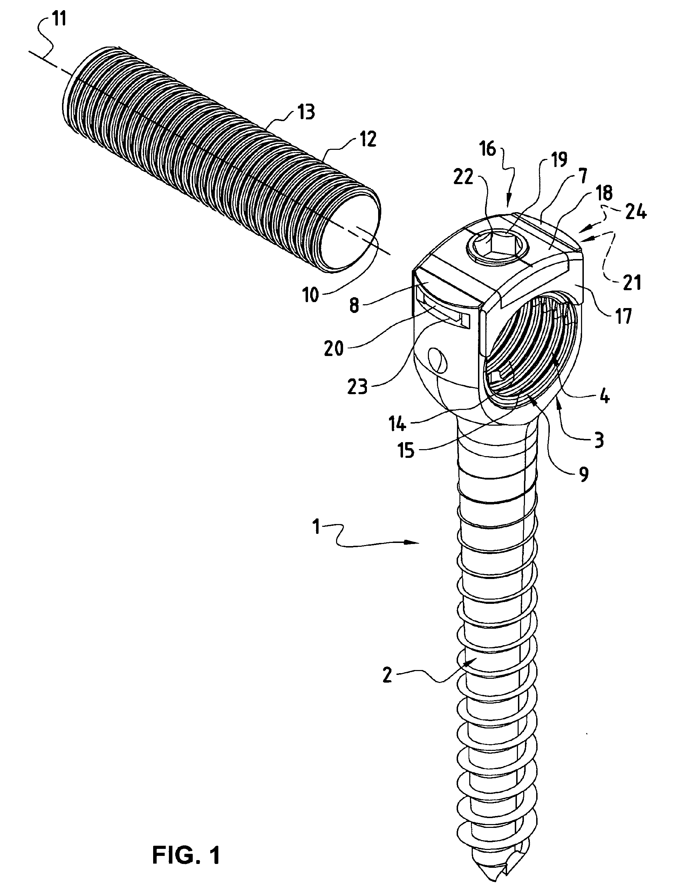

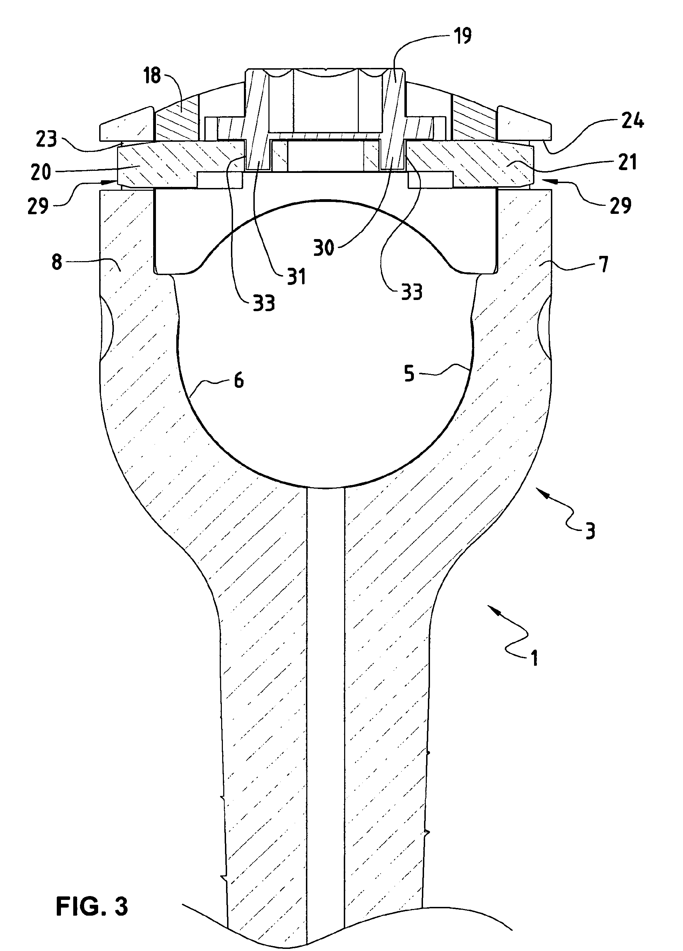

Wie aus Figur 1 ersichtlich ist, besteht die Pedikelschraube 1 aus einem mit einem Gewinde versehenen Einschraubteil 2, mit welchem die Pedikelschraube 1 in bekannter Weise in einen Wirbelkörper einer Wirbelsäule eines Patienten eingeschraubt werden kann. An diesem Einschraubteil 2 ist ein Kopfteil 3 angebracht, welcher mit einer U-förmigen Ausnehmung 4 versehen ist. Diese U-förmige Ausnehmung wird durch die inneren Oberflächen 5, 6 (Fig. 3) zweier Schenkel 7, 8 gebildet, welche am Einschraubteil 2 befestigt sind. Die beiden inneren Oberflächen 5 und 6 dieser beiden Schenkel werden durch eine Grundfläche 9 miteinander verbunden, welche Grundfläche 9 der Form des in diese U-förmige Ausnehmung 4 einzusetzenden Stabes 10 angepasst ist, welcher in Figur 1 ausserhalb der Pedikelschraube 1 dargestellt ist.

Dieser Stab 10 ist mit quer zur Stabachse 11 verlaufenden Rippen 12 und Rillen 13 ausgestattet. Die Grundfläche 9 der U-förmigen Ausnehmung 4 ist ebenfalls mit Rippen 14 und Rillen 15 versehen, die den Rippen 12 und Rillen 13 des Stabes 10 entsprechend sind. Im in die U-förmige Ausnehmung 4 eingesetzten Zustand des Stabes 10 befinden sich die Rippen 12 des Stabes 10 in den entsprechenden Rillen 15 der U-förmigen Ausnehmung 4, während die Rippen 14 der U-förmigen Ausnehmung 4 in die Rillen 13 des Stabes 10 eindringen. Dadurch erhält man eine formschlüssige Verbindung, ein Verschieben des Stabes 10 bezüglich der Pedikelschraube 1 in Richtung der Stabachse 11 ist nicht möglich.

Auf die in der Figur 1 dargestellten Pedikelschraube 1 ist eine Verschlusseinrichtung 16 aufgesetzt. Diese Verschlusseinrichtung 16 besteht aus mehreren zu einer Einheit zusammengesetzten Einzelteilen, nämlich einem Tragelement 17, einem Abschlussdeckel 18, einem drehbaren Teil 19 und zwei Schiebeteilen 20 und 21, diese Einzelteile werden später noch im Detail beschrieben. Über den drehbaren Teil 19 lassen sich die beiden Schiebeteile 20 und 21 verschieben, wodurch die Verschlusseinrichtung 16 im auf die Pedikelschraube 1 aufgesetzten Zustand verriegelt werden kann, der in die Pedikelschraube 1 eingesetzte Stab 10 wird dadurch in optimaler Weise gehalten. Zur Bedienung des drehbaren Teils 19 ist dieser mit einer profilierten Vertiefung 22 ausgestattet, in welche ein mit einer entsprechenden profilierten Form versehenes, bekanntes nicht dargestelltes Drehwerkzeug eingesteckt werden kann, wodurch die Veschlusseinrichtung 16 durch Verdrehen dieses Drehwerkszeugs vom entriegelten Zustand in den verriegelten Zustand und umgekehrt gebracht werden kann. Die beiden Schenkel 7 und 8 des Kopfteiles 3 der Pedikelschraube 1 sind zur Aufnahme der beiden Schiebeteile 20 und 21 jeweils mit einer Ausnehmung 23, 24 ausgestattet.

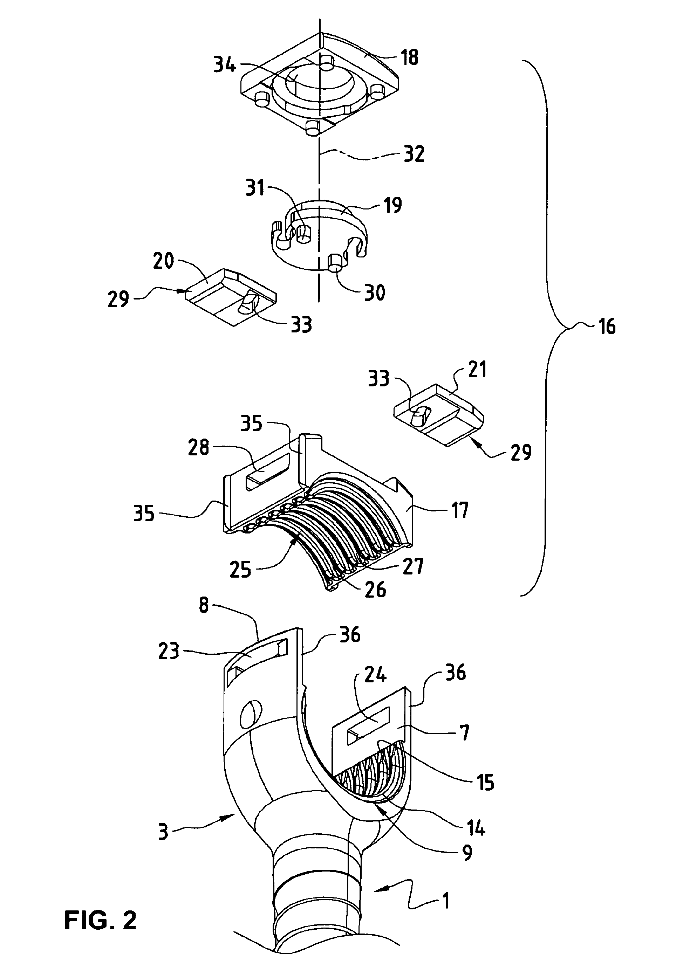

Figur 2 zeigt den Kopfteil 3 der Pedikelschraube 1, mit den beiden Schenkeln 7 und 8, die durch die Grundfläche 9 miteinander verbunden sind, welche Grundfläche 9 mit den entsprechenden Rippen 14 und Rillen 15 ausgestattet ist. In den beiden Schenkeln 7 und 8 sind die beiden Ausnehmungen 23 und 24 angebracht, in welche die beiden Schiebeteile 20 bzw. 21 eingeschoben werden können.

Das Tragelement 17 ist mit einer Oberfläche 25 versehen, welche, wie aus Figur 1 ersichtlich ist, im auf die Pedikelschraube 1 aufgesetzten Zustand der Verschlusseinrichtung 16 gegen den in die Pedikelschraube 1 eingesetzten Stab 10 gerichtet ist. Diese Oberfläche 25 weist eine der Staboberfläche angepasste Form auf, sie ist zudem ebenfalls mit Rippen 26 und Rillen 27 versehen, die den Rippen 14 und den Rillen 15 der Grundfläche 9 des Kopfteils entsprechen und mit diesem fluchtend sind, sodass die Rippen 12 und die Rillen 13 des Stabes 10 vom Kopfteil 3 und der Verschlusseinrichtung 16 vollumfänglich umschlossen sind, wodurch eine optimale formschlüssige Fixierung des Stabes 10 in dieser Pedikelschraube 1 erreicht wird.

In das Tragelement 17 können die beiden Schiebeteile 20 und 21 in entsprechende, nicht dargestellte Führungen eingelegt werden, hierzu ist das Tragelement 17 auf beiden Seiten mit jeweils einer Öffnung 28 versehen, die beiden Schiebeteile 20 und 21 lassen sich somit entlang dieser nicht dargestellten Führungen gegeneinander und voneinander weg verschieben, wobei im auseinander geschobenen Zustand diese beiden Schiebeteile 20, 21 mit den beiden voneinander abgewandten Endbereichen 29 durch die Öffnungen 28 hindurch ragen und vorstehend sind.

Auf die beiden in das Tragelement 17 verschiebbar eingesetzten Schiebeteile 20 und 21 wird der drehbare Teil 19 aufgesetzt. Dieser drehbare Teil 19 ist an der den beiden Schiebeteilen 20 und 21 zugewandten Fläche mit jeweils zwei Zapfen 30 und 31 ausgestattet, die parallel zur Achse 32 ausgerichtet sind, um welche Achse 32 der drehbare Teil 19 drehbar ist. Die beiden Zapfen 30 und 31 ragen jeweils in eine an jedem Schiebeteil 20 und 21 angebrachten Steuerkurve 33 hinein, welche Steuerkurve 33 als im jeweiligen Schiebeteil 20 bzw. 21 angebrachte schlitzförmige Ausnehmung ausgestaltet ist.

Auf den drehbaren Teil 19 aufgesetzt ist der Abschlussdeckel 18, der mit einer Bohrung 34 ausgestattet ist, in welcher der drehbare Teil 19 drehbar gelagert ist. Der Abschlussdeckel 18 lässt sich in bekannter Weise mit dem Tragelement 17 fest verbinden, beispielsweise über Presszapfen oder in anderer bekannter geeigneter Art. Die derart zusammengebaute Verschlusseinrichtung 16 lässt sich bei in die Pedikelschraube eingesetztem Stab auf die Pedikelschraube 1 aufsetzen, wie dies in Figur 1 dargestellt ist. Hierzu weist das Tragelement an seinen Eckbereichen jeweils eine Führungsrippe 35 auf, mittels welcher das Tragelement 17 und somit die Verschlusseinrichtung 16 geführt auf den Kopfteil 3 aufsetzen, wobei der Kopfteil 3 entsprechend mit Führungsflächen 36 ausgestattet ist.

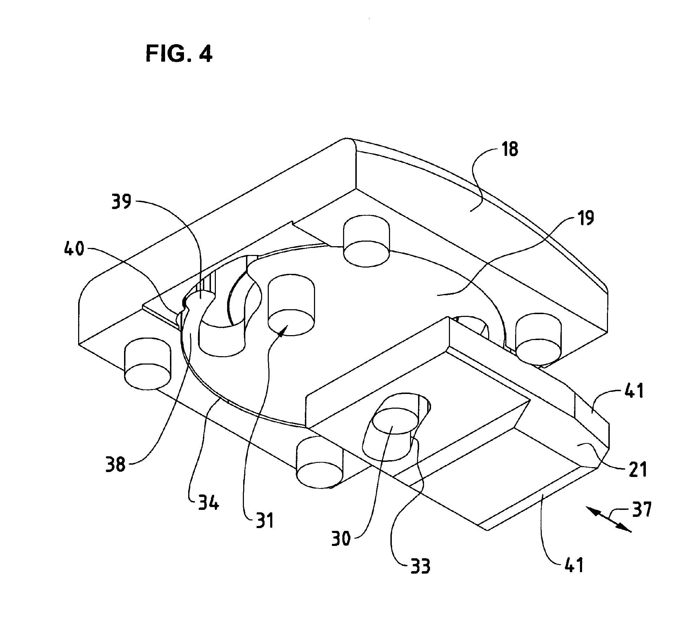

Aus Figur 4 ist der Abschlussdeckel 18, der darin eingesetzte drehbare Teil 19 und einer der beiden Schiebeteile 21 im zusammengebauten Zustand dargestellt, wodurch das Zusammenwirken verdeutlicht wird. Durch Verdrehen des drehbaren Teils 19 im Abschlussdeckel 18 wird über den Zapfen 30 und die im Schiebeteil 21 angebrachte Steuerkurve 33 der Schiebeteil 21 in Richtung des Pfeils 37 verschoben. Entsprechend wird über den Zapfen 31 der nicht dargestellte Schiebeteil 20 ebenfalls in Richtung des Pfeils 37 verschoben, man erreicht somit ein Verschieben der beiden Schiebeteile 20 und 21 von einer gegeneinander geschobenen Position in eine auseinander geschobene Position.

Figur 3 zeigt die Verschlusseinrichtung 16, die auf die Pedikelschraube 1 aufgesetzt ist. Durch Verdrehen des drehbaren Teils 19 wurden nach dem Aufsetzen der Verschlusseinrichtung 16 auf den Kopfteil 3 der Pedikelschraube 1 die beiden Schiebeteile 20 und 21 über die am drehbaren Teil 19 angebrachten Zapfen 30 und 31 und die Steuerkurven 33 in den beiden Schiebeteilen 20 und 21 in die auseinander geschobene Position gefahren. Die einander abgewandten Endbereiche 29 der beiden Schiebeteile 20 und 21 wurden dabei in die Ausnehmungen 23 und 24 eingeschoben, die am jeweiligen Schenkel 7 bzw. 8 des Kopfteils 3 der Pedikelschraube 1 angebracht sind. Dadurch wird die Verschlusseinrichtung 16 im Kopfteil 3 der Pedikelschraube 1 verriegelt, ein in den Kopfteil 3 der Pedikelschraube 1 eingesetzer Stab würde somit formschlüssig festgehalten.

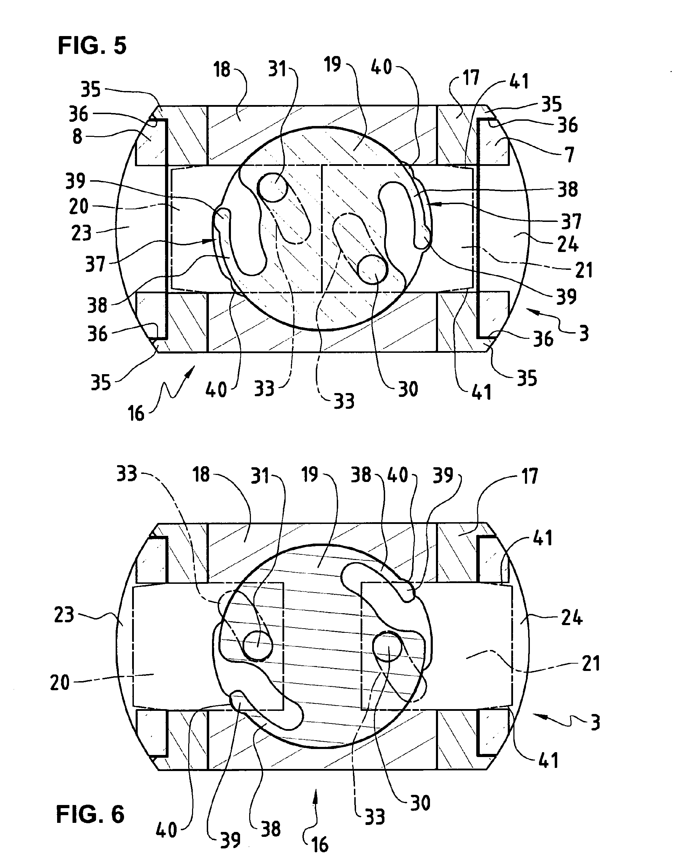

Figur 5 zeigt die beiden Schiebeteile 20 und 21 in der gegeneinander geschobenen Position, dies bedeutet, dass in dieser Position die Verschlusseinrichtung 16 auf den Kopfteil 3 der Pedikelschraube aufgesetzt werden kann. Die Führungsrippen 35 werden hierbei durch die Führungsflächen 36 der beiden Schenkel 7 und 8 geführt. Die beiden Schiebeteile 20 und 21 sind soweit zurückgezogen, dass sie zwischen die beiden Schenkel 7 und 8 einfahren können.

Der drehbare Teil 19 ist mit Einrastelementen 37 ausgestattet. Diese Einrastelemente 37 bestehen jeweils aus einem federnden Arm 38, dessen eines Ende am drehbaren Teil 19 befestigt ist. Am federnden freien Ende jedes federnden Armes 38 ist ein Nocken 39 angebracht.

Wenn die Verschlusseinrichtung 16 auf den Kopfteil 3 aufgesetzt ist, befinden sich die Schiebeteile 20 und 21 auf der Höhe der Ausnehmungen 23 und 24, die an den beiden Schenkeln 7 und 8 des Kopfteils 3 angebracht sind. Durch Verdrehen des drehbaren Teils 19 werden die beiden Schiebeteile 20 und 21 in die auseinander geschobene Position gebracht, was durch das Gleiten der beiden Zapfen 30 und 31 in den Steuerkurven 33 ausgeführt wird, wie dies aus Figur 6 ersichtlich ist. Die Verschlusseinrichtung 16 befindet sich somit in der bezüglich des Kopfteils 3 verriegelten Position. In diese Position gelangen die beiden Nocken 39 der federnden Arme 38 in eine am Abschlussdeckel 18 angebrachte Ausnehmung 40 und rasten hier ein. In dieser Drehstellung des drehbaren Teils 19 wird dieser somit in dieser Position gehalten, ein selbstständiges ungewolltes Zurückdrehen des drehbaren Teils 19 wird unterbunden, somit ist die verriegelte Position der Verschlusseinrichtung 16 bezüglich des Kopfteils 3 gewährleistet. Dieser Einrastvorgang lässt auch die die Verschlusseinrichtung bedienende Person spüren, dass die Einrastposition erreicht ist, wodurch angezeigt wird, dass die Verschlusseinrichtung optimal verriegelt ist. Durch die an den Schiebeteilen 20 und 21 angebrachten Anschrägungen 41, wie diese insbesondere auch in Figur 4 ersichtlich sind, ist gewährleistet, dass das Einfahren der Schiebeteile 20 und 21 in die jeweilige Ausnehmung 23 bzw. 24 der beiden Schenkel 7 und 8 in optimaler Weise möglich. In Figur 4 ist auch einer der beiden federnden Arme 38 mit dem Nocken 39 ersichtlich, welcher am drehbaren Teil 19 befestigt ist, sowie die in der Bohrung 34 des Abschlussdeckels 18 angebrachte Ausnehmung 40, in welche der Nocken 39 einrasten kann.

Mit dieser Verschlusseinrichtung zu der entsprechend ausgestatteten Pedikelschraube wird eine sehr einfache Handhabung beim Einsetzen und Fixieren des Stabes erreicht, es wird auch erreicht, dass der Stab in optimaler Weise in der Pedikelschraube formschlüssig gehalten wird, durch die Einrastelemente wird auch sicher gestellt, dass sich die Verschlusseinrichtung nicht selbstständig lösen kann. Die Pedikelschraube und die Verschlusseinrichtung werden in vorteilhafter Weise aus einer Titanlegierung hergestellt.

## Patentansprüche

1. Pedikelschraube mit einer Verschlusseinrichtung (16) zur Befestigung eines Stabes (10) zur Stabilisierung der Wirbelsäule, umfassend einen Einschraubteil (2), einen am Einschraubteil (2) angebrachten Kopfteil (3), eine im Kopfteil (3) angeordnete U-förmige Ausnehmung (4), die durch die inneren Oberflächen (5, 6) zweier Schenkel (7, 8) und einer die beiden inneren Oberflächen (5, 6) verbindenden Grundfläche (9) gebildet ist, in welche Ausnehmung (4) der Stab (10) einsetzbar und durch die Verschlusseinrichtung (16) gehalten ist, welcher Stab (10) mit im wesentlichen quer zur Stabachse (11) verlaufenden Rippen (12) und Rillen (13) und welche Grundfläche mit diesen entsprechenden Rippen (14) und Rillen (15) ausgestattet sind, **dadurch gekennzeichnet, dass** die Verschlusseinrichtung (16) ein Tragelement (17) umfasst, welches zwischen die beiden Schenkel (7, 8) einsetzbar ist, dass am Tragelement (17) zwei Schiebeteile (20, 21) verschiebbar angeordnet sind, welche über einen Antriebsmechanismus von einer gegeneinander geschobenen Position in eine auseinander geschobene Position bringbar sind, in welcher auseinandergeschobenen Position die beiden voneinander abgewandten Endbereiche (29) der beiden Schiebeteile (20, 21) bei zwischen die Schenkel (7, 8) eingesetztem Zustand des Tragelementes (17) in jeweils eine Ausnehmung (23, 24) der beiden Schenkel (7, 8) hineinragen und die Verschlusseinrichtung (16) mit der Pedikelschraube verriegelt ist.

2. Pedikelschraube mit einer Verschlusseinrichtung nach Anspruch 1, **dadurch gekennzeichnet, dass** der Antriebsmechanismus aus einem drehbaren Teil (19) besteht, der im Tragelement (17) um eine senkrecht zur Verschieberichtung der beiden Schiebeteile (20, 21) stehende Achse (32) drehbar angeordnet ist, welcher mit zwei parallel zur Achse (32) ausgerichtete Zapfen (30, 31) ausgestattet ist, welche jeweils mit einer an jedem Schiebeteil (20, 21) angebrachten Steuerkurve (33) zusammenwirken.

3. Pedikelschraube mit einer Verschlusseinrichtung nach Anspruch 2, **dadurch gekennzeichnet, dass** die Schiebeteile (20, 21) und der drehbare Teil (19) in das Tragelement (17) eingelegt sind und dass auf das Tragelement (17) ein Abschlussdeckel (18) aufgesetzt und mit diesem verbunden ist, der mit einer Bohrung (34) versehen ist, in welcher der drehbare Teil (19) drehbar gelagert ist.

4. Pedikelschraube mit einer Verschlusseinrichtung nach Anspruch 2 oder 3, **dadurch gekennzeichnet, dass** zwischen drehbarem Teil (19) und Tragelement (17) Einrastelemente (37) angeordnet sind, welche die beiden Schiebeteile (20, 21) in der auseinandergeschobenen Position in einer Einrastlage festhalten.

5. Pedikelschraube mit einer Verschlusseinrichtung nach Anspruch 4, **dadurch gekennzeichnet, dass** die Einrastelemente (37) jeweils aus einem federnden Arm (38) gebildet sind, dessen eines Ende am drehbaren Teil (19) befestigt ist und an dessen federndem freien Ende ein Nocken (39) angebracht ist, welcher in der Einrastlage in jeweils eine entsprechende am Tragelement (17) bzw. am Abschlussdeckel (18) angebrachte Ausnehmung einrastet.

6. Pedikelschraube mit einer Verschlusseinrichtung nach einem der Ansprüche 2 bis 5, **dadurch gekennzeichnet, dass** der drehbare Teil (19) mit einer profilierten Vertiefung (22) ausgestattet ist, in welche ein mit einer entsprechenden profilierten Form versehenes Drehwerkzeug einsteckbar ist.

7. Pedikelschraube mit einer Verschlusseinrichtung nach einem der Ansprüche 1 bis 6, **dadurch gekennzeichnet, dass** die voneinander abgewandten Endbereiche (29) der Schiebeteile (20, 21) mit Anschrägungen (41) versehen sind.

8. Pedikelschraube mit einer Verschlusseinrichtung nach einem der Ansprüche 1 bis 7, **dadurch gekennzeichnet, dass** die dem Stab (10) zugewandte Oberfläche (25) des Tragelements (17) eine der Staboberfläche angepasste Form aufweist und dass diese Oberfläche (25) mit entsprechenden Rippen (26) und Rillen (27) ausgestattet ist.

9. Pedikelschraube mit einer Verschlusseinrichtung nach einem der Ansprüche 1 bis 8, **dadurch gekennzeichnet, dass** das Tragelement (17) an seinen Eckbereichen mit Führungsrippen (35) ausgestattet ist, mittels welchen das Tragelement (17) geführt auf den Kopfteil (3), der mit Führungsflächen (36) versehen ist, aufsetzbar ist.

10. Pedikelschraube mit einer Verschlusseinrichtung nach einem der Ansprüche 1 bis 9, **dadurch gekennzeichnet, dass** sie einen in die U-förmige nehmung der Pedikelschraube einsetzbaren, und durch die Verschlusseinrichtung gehaltenen Stab (10) aufweist, der aus einem elastischen Material, insbesondere aus einem biokompatiblen Kunststoff auf der Basis von Polyurethan, gebildet ist.

## Claims

1. Pedicle screw with a closure device (16) for securing a rod (10) for stabilization of the vertebral column, comprising a screw-in part (2), a head part (3) provided on the screw-in part (2), a U-shaped recess (4) disposed in the head part (3), which recess is formed by the inner surfaces (5, 6) of two arms (7, 8) and a bottom surface (9) connecting the two inner surfaces (5, 6), in which recess (4) the rod (10) is insertable and is held by the closure device (16), which rod (10) is provided with ridges (12) and grooves (13) running substantially transversely with respect to the rod axis (11), and which bottom surface is provided with corresponding ridges (14) and grooves (15), **characterized in that** the closure device (16) comprises a support element (17), which is insertable between the two arms (7, 8), **in that** two sliding parts (20, 21) are disposed in a displaceable way on the support element (17), which sliding parts are able to be brought, via a movement mechanism, from a position of being pushed against each other into a position of being pushed apart, in which pushed-apart position the two end regions (29), facing away from each other, of the two sliding parts (20, 21) each project into a recess (23, 24) of the two arms (7, 8) in the state of the support element (17) being inserted between the arms (7, 8), and the closure device (16) is locked with the pedicle screw.

2. Pedicle screw with a closure device according to claim 1, **characterized in that** the movement mechanism consists of a rotatable part (19), which is disposed in the support element (17) in a way rotatable about an axis (32) perpendicular to the direction of displacement of the two sliding parts (20, 21), which rotatable part is provided with two pins (30, 31) aligned parallel to the axis (32), which pins each co-operate with a cam device (33) provided on each sliding part (20, 21).

3. Pedicle screw with a closure device according to claim 2, **characterized in that** the sliding parts (20, 21) and the rotatable part (19) are inserted in the support element (17), and **in that** a cover (18) is placed on the support element (17) and is connected to the latter, which cover is provided with a bore (34) in which the rotatable part (19) is rotatably borne.

4. Pedicle screw with a closure device according to claim 2 or 3, **characterized in that** disposed between rotatable part (19) and support element (17) are engagement elements (37), which keep the two sliding parts (20, 21) in an engaged state in the pushed-apart position.

5. Pedicle screw with a closure device according to claim 4, **characterized in that** the engagement elements (37) are each made up of a resilient arm (38), the one end of which is attached to the rotatable part (19), and on the resilient free end of which a protrusion (39) is provided which, in the engaged state, engages in each case in a corresponding recess provided on the support element (17) or respectively on the cover (18).

6. Pedicle screw with a closure device according to one of the claims 2 to 5, **characterized in that** the rotatable part (19) is provided with a contoured cavity (22), into which a turning tool having a correspondingly contoured shape is insertable.

7. Pedicle screw with a closure device according to one of the claims 1 to 6, **characterized in that** the end regions (29), remote from each other, of the sliding parts (20, 21) are provided with bevels (41).

8. Pedicle screw with a closure device according to one of the claims 1 to 7, **characterized in that** the surface (25) of the support element (17) turned toward the rod (10) has a shape adapted to the rod surface, and **in that** this surface (25) is provided with corresponding ridges (26) and grooves (27).

9. Pedicle screw with a closure device according to one of the claims 1 to 8, **characterized in that** the support element (17) is provided with guide ribs (35) at its corner regions by means of which the support element (17) is placeable in a guided way on the head part (3), which is provided with guide surfaces (36).

10. Pedicle screw with a closure device according to one of the claims 1 to 9, **characterized in that** it has a rod (10), insertable in the U-shaped recess of the pedicle screw and held by the closure device, and made of an elastic material, in particular a polyurethane-based biocompatible plastic.

## Revendications

1. Vis pédiculaire dotée d'un dispositif de fermeture (16) pour la fixation d'une tige (10) pour stabiliser la colonne vertébrale, comprenant une partie de vissage (2), une partie de tête (3) prévue sur la partie de vissage (2), un évidement en forme de U (4) ménagé dans la partie de tête (3), ledit évidement étant constitué par les surfaces internes (5, 6) de deux bras (7, 8) et une surface de base (9) reliant les deux surfaces internes (5, 6), évidement (4) dans lequel la tige (10) est susceptible d'être insérée et retenue par ledit dispositif de fermeture (16), ladite tige (10) étant pourvue de nervures (12) et de rainures (13) s'étendant sensiblement transversalement par rapport a l'axe de tige (11), et ladite surface de base étant pourvue de nervures (14) et de rainures (15) correspondantes, **caractérisée en ce que** ledit dispositif de fermeture (16) comprend un élément de support (17) susceptible d'être inséré entre les deux bras (7, 8), **en ce que** deux pièces coulissantes (20, 21) sont agencées de manière déplaçable sur ledit élément de support (17), lesdites pièces coulissantes pouvant être amenées par un mécanisme d'entraînement, à partir d'une position où ces dernières sont approchées l'une vers l'autre, vers une position où ces dernières sont éloignées l'une de l'autre, dans ladite position où ces dernières sont éloignées l'une de l'autre, les deux régions d'extrémité (29), orientées vers des directions éloignées l'une de l'autre, desdites deux pièces coulissantes (20, 21) faisant saillie chacune dans un évidement (23, 24) des deux bras (7, 8) dans l'état où ledit élément de support (17) est inséré entre lesdits bras (7, 8), et **en ce que** ledit dispositif de fermeture (16) est verrouillé par ladite vis pédiculaire.

2. Vis pédiculaire dotée d'un dispositif de fermeture selon la revendication 1, **caractérisée en ce que** ledit mécanisme d'entraînement est constituée d'une pièce rotative (19) qui est agencée dans ledit élément de support (17) de manière rotative autour d'un axe (32) perpendiculaire à la direction de déplacement desdites deux pièces coulissantes (20, 21), ladite pièce rotative étant pourvue de deux ergots (30, 31) alignés parallèlement audit axe (32), lesdits ergots coopérant chacun avec une courbe de commande (33) prévue sur chaque pièce coulissante (20, 21).

3. Vis pédiculaire dotée d'un dispositif de fermeture selon la revendication 2, **caractérisée en ce que** lesdites pièces coulissantes (20, 21) et ladite pièce rotative (19) sont insérées (intercalées) dans ledit élément de support (17), et **en ce qu'**un couvercle (18) est placé sur ledit élément de support (17) et est relié à ce dernier, ledit couvercle étant pourvu d'un alésage (34) dans lequel ladite pièce rotative (19) est montée en rotation.

4. Vis pédiculaire dotée d'un dispositif de fermeture selon l'une quelconque des revendications 2 ou 3, **caractérisée en ce que** des éléments d'enclenchement (37) sont agencés entre ladite pièce rotative (19) et ledit élément de support (17), et que lesdits éléments d'enclenchement maintiennent les deux pièces coulissantes (20, 21) dans un état enclenché dans ladite position où ces dernières sont éloignées l'une de l'autre.

5. Vis pédiculaire dotée d'un dispositif de fermeture selon la revendication 4, **caractérisée en ce que** lesdits éléments d'enclenchement sont chacun constitués d'un bras élastique (38) dont une extrémité est attachée à ladite pièce rotative (19) et dont l'extrémité libre élastique est pourvue d'une saillie (39) qui, dans l'état enclenché, vient s'enclencher à chaque fois dans un évidement correspondant prévu sur ledit élément de support (17) ou respectivement sur ledit couvercle (18).

6. Vis pédiculaire dotée d'un dispositif de fermeture selon l'une quelconque des revendications 2 à 5, **caractérisée en ce que** ladite pièce rotative (19) est pourvue d'un évidement profilé (22) dans lequel est susceptible d'être inséré un outil à tourner dont la forme est profilée de manière correspondante.

7. Vis pédiculaire dotée d'un dispositif de fermeture selon l'une quelconque des revendications 1 à 6, **caractérisée en ce que** lesdites régions d'extrémité (29), éloignées l'une de l'autre, desdites pièces coulissantes (20, 21) sont pourvues de chanfreins (41).

8. Vis pédiculaire dotée d'un dispositif de fermeture selon l'une quelconque des revendications 1 à 7, **caractérisée en ce que** la surface (25), tournée vers la tige (10), dudit élément de support (17) a une forme adaptée à la surface de ladite tige, et **en ce que** ladite surface (25) est pourvue de nervures (26) et de rainures (27) correspondantes.

9. Vis pédiculaire dotée d'un dispositif de fermeture selon l'une quelconque des revendications 1 à 8, **caractérisée en ce que** ledit élément de support (17) est pourvu, au niveau de ses régions de coin, de nervures de guidage (35) par lesquelles ledit élément de support (17) est susceptible d'être placé de manière guidée sur ladite partie de tête (3) qui est pourvue de surfaces de guidage (36).

10. Vis pédiculaire dotée d'un dispositif de fermeture selon l'une quelconque des revendications 1 à 9, **caractérisée en ce qu'**elle comporte une tige (10) susceptible d'être insérée dans ledit évidement en forme de U de la vis pédiculaire et maintenue par ledit dispositif de fermeture, ladite tige étant fabriquée d'un matériau élastique, notamment d'une matière plastique biocompatible à base de polyuréthane.
